# EUROPÄISCHE PATENTANMELDUNG

(11) **EP 2 236 163 A2**
(43) Veröffentlichungstag der Anmeldung: **06.10.2010**
(21) Anmeldenummer: 10155868.2
(22) Anmeldetag: 09.03.2010
(51) Int. Cl.: A61L 31/02, A61L 31/08, A61L 31/16

(54) **Implantat aus einem biokorrodierbaren metallischen Werkstoff mit einer nanopartikel-haltigen Silanbeschichtung und dazugehöriges Herstellungsverfahren**

(30) Priorität: 02.04.2009 DE 102009002153
(71) Anmelder: Biotronik VI Patent AG, 6341 Baar (CH)
(72) Erfinder: Hausbeck, Timo, 91052, Erlangen (DE); Rzany, Alexander, 90449, Nürnberg (DE)
(74) Vertreter: Lindner-Vogt, Karin L.

(57) **Zusammenfassung**

Die Erfindung betrifft ein Implantat aus einem biokorrodicrbarcn metallischen Werkstoff mit einer passivierenden Beschichtung aus einer nanopartikel-haltigen Silanbeschichtung sowie ein dazugehöriges Verfahren zur Herstellung des Implantats. Das Verfahren umfasst dazu die Schritte:
(i) Bereitstellen eines Rohlings für das Implantat bestehend aus dem biokorrodierbaren metallischen Werkstoff; und
(ii) Beschichten des Rohlings mit einer homogen-kolloidalen Lösung aus
A) einem oder mehreren Bis-Silanen der Formel (1):

(RO)₃-Si-X-Si(OR)₃ (1)

wobei R einzeln ausgewählt für C₁-C₁₀-Alkyl oder C₂-C₁₀-Acyl steht; und
X eine substituierte oder unsubstituierte C₁-C₂₀-Alkandiylgruppe oder eine substituierte oder unsubstituierte C₁-C₂₀-Heteroalkandiylgruppe mit 1 bis 5 Heteroatomen ausgewählt aus der Gruppe O, N und S ist; und
B) Siliziumdioxid-Nanopartikeln mit einem mittleren Partikeldurchmesser im Bereich von 10 nm bis 1 µm

in Wasser, Ethanol oder einem Gemisch derselben, wobei die Lösung 1 bis 20 Vol.% Bis-Silan enthält und die Konzentration der Siliziumdioxid-Nanopartikel in der Lösung im Bereich von 1 bis 100 ppm liegt.

## Beschreibung

Die Erfindung betrifft ein Implantat aus einem biokorrodierbaren metallischen Werkstoff mit einer passivierenden Beschichtung aus einer Siliziumverbindung sowie ein dazugehöriges Verfahren zur Herstellung des Implantats.

### Hintergrund der Erfindung

Medizinische Implantate unterschiedlichster Zweckbestimmung sind in großer Vielfalt aus dem Stand der Technik bekannt. Häufig ist nur ein zeitweiliger Verbleib des Implantats im Körper zur Erfüllung des medizinischen Zweckes erforderlich. Implantate aus permanenten Werkstoffen, also Werkstoffen, die im Körper nicht abgebaut werden, sind in vielen Anwendungen wieder zu entfernen, da es mittel- und langfristig auch bei hoher Biokompatibilität zu Abstoßungsreaktionen des Körpers kommen kann.

Ein Ansatz zur Vermeidung eines dauerhaften Verbleibs des Implantats im Körper bzw. weiteren chirurgischen Eingriffs zur Entfernung desselben besteht nun darin, das Implantat ganz oder in Teilen aus einem biokorrodierbaren Werkstoff zu formen. Unter Biokorrosion werden mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert, wobei geringe Rückstände tolerierbar sind.

Biokorrodierbare Werkstoffe wurden unter anderem auf Basis von Polymeren synthetischer Natur oder natürlichen Ursprungs entwickelt. Die Materialeigenschaften, aber auch teils die Biokompatibilität der Abbauprodukte der Polymere, limitieren den Einsatz jedoch deutlich. So müssen beispielsweise orthopädische Implantate häufig hohen mechanischen Beanspruchungen standhalten und vaskuläre Implantate, z. B. Stents, je nach Design sehr speziellen Anforderungen an E-Modul, Bruchdehnung und Formbarkeit genügen.

Ein aussichtsreicher Ansatz zur Lösung des Problems liegt in der Verwendung biokorrodierbarer Metalllegierungen. So wird in DE 197 31 021 A1 vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium > 90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stent, eignet. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Die relativ rasche Biokorrosion der Magnesiumlegierungen limitiert den Einsatz.

Sowohl die Grundlagen der Magnesium-Korrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen einer Magnesiumlegierung einen leicht erhöhten Korrosionswiderstand in Meerwasser verleiht.

Ein Ansatzpunkt sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden unter dem Gesichtpunkt eines technischen Einsatzes des Formkörpers - jedoch nicht medizin-technischen Einsatzes in biokorrodierbaren Implantaten in physiologischer Umgebung - entwickelt und optimiert. Diese bekannten Verfahren umfassen: das Aufbringen von Polymeren, das Erzeugen einer Emaille, die chemische Konversion der Oberfläche, Heißgasoxidation, Anodisieren, Plasmaspritzen, Laserstrahl-Umschmelzen, PVD-Verfahren und Ionenimplantation.

Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Inhibierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung korrosiver Prozesse liegen. Schon aus diesem Grunde eignen sich die meisten bekannten Verfahren zur Erzeugung einer Korrosionsschutzschicht nicht. Ferner müssen für einen medizin-technischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein. Schließlich dürften bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Nahtmaterial oder Clips im Gebrauch mechanisch verformt, so dass der Teilprozess der Spannungsriss-Korrosion beim Abbau dieser Formkörper erhebliche Bedeutung haben dürfte.

DE 101 63 106 A1 sieht vor, den Magnesiumwerkstoff durch Modifikation mit Halogeniden in seiner Korrosivität zu verändern. Der Magnesiumwerkstoff soll zur Herstellung medizinischer Implantate verwendet werden. Bevorzugt ist als Halogenid ein Fluorid. Die Modifikation des Werkstoffs wird durch Zulegieren salzförmiger Halogenverbindungen erreicht. Es wird demnach die Zusammensetzung der Magnesiumlegierung durch Zugabe der Halogenide verändert, um die Korrosionsrate zu mindern. Dementsprechend wird der gesamte, aus einer solchen modifizierten Legierung bestehende Formkörper ein verändertes Korrosionsverhalten besitzen. Durch das Zulegieren können jedoch weitere Werkstoffeigenschaften beeinflusst werden, die bei der Verarbeitung von Bedeutung sind oder auch die mechanischen Eigenschaften des aus dem Werkstoff entstehenden Formkörpers prägen.

Ferner sind Beschichtungen für Implantate aus nicht-biokorrodierbaren also permanenten Werkstoffen bekannt, die auf Organosiliziumverbindungen basieren. So beschreibt beispielsweise DE 699 12 951 T2 eine Zwischenschicht aus einem funktionalisierten Silikonpolymer, wie Siloxanen oder Polysilanen. US 2004/0236399 A1 offenbart einen Stent mit einer Silanschicht, die von einer weiteren Schicht bedeckt wird.

DE 10 2006 038 231 A1 beschreibt ein Implantat aus einem biokorrodierbaren metallischen Werkstoff mit einer Beschichtung aus einer Organosiliziumverbindung. Die Beschichtung kann unter anderem unter Verwendung eines Bis-Silans hergestellt werden.

### Zusammenfassung der Erfindung

Der vorliegenden Erfindung liegt die Aufgabe zugrunde, ein oder mehrere der zuvor geschilderten Probleme zu lösen oder zumindest zu mindern. Insbesondere soll eine Beschichtung für ein Implantat aus einem biokorrodierbaren Werkstoff bereitgestellt werden, die eine temporäre Inhibition bewirkt, aber nicht eine vollständige Unterbindung der Korrosion des Werkstoffs in physiologischer Umgebung.

Diese Aufgabe wird nach einem ersten Aspekt der Erfindung durch das erfindungsgemäße Verfahren zur Herstellung eines Implantats aus einem biokorrodierbaren metallischen Werkstoff mit einer nanopartikel-haltigen Silanbeschichtung gelöst. Das Verfahren umfasst dazu die Schritte:
(i) Bereitstellen eines Rohlings für das Implantat bestehend aus dem biokorrodierbaren metallischen Werkstoff; und
(ii) Beschichten des Rohlings mit einer homogen-kolloidalen Lösung aus
   A) einem oder mehreren Bis-Silanen der Formel (1):

      (RO)₃-Si-X-Si(OR)₃ (1)

      wobei R einzeln ausgewählt für C₁-C₁₀-Alkyl oder C₂-C₁₀-Acyl steht; und
      X eine substituierte oder unsubstituierte C₁-C₂₀-Alkandiylgruppe oder eine substituierte oder unsubstituierte C₁-C₂₀-Heteroalkandiylgruppe mit 1 bis 5 Heteroatomen ausgewählt aus der Gruppe O, N und S ist; und
   B) Siliziumdioxid-Nanopartikeln mit einem mittleren Partikeldurchmesser im Bereich von 10 nm bis 1 µm
in Wasser, Ethanol oder einem Gemisch derselben, wobei die Lösung 1 bis 20 Vol.% Bis-Silan enthält und die Konzentration der Siliziumdioxid-Nanopartikel in der Lösung im Bereich von 1 bis 100 ppm liegt.

Es hat sich nun gezeigt, dass durch Kombination eines oder mehrerer Bis-Silane der oben genannten Formel mit SiO₂-Nanopartikeln eine für die angestrebten Zwecke vorteilhafte korrosionshemmende Beschichtung auf dem Grundkörper aus dem biokorrodierbaren metallischen Werkstoff hergestellt werden kann. Vermutlich werden durch die Anwesenheit von SiO₂-Nanopartikeln in der Beschichtung Hydroxidionen an der Substratoberfläche unter Bildung eines passivierenden Silikats gebunden. Dadurch werden kathodische Reaktionen bei der Korrosion der Werkstoffe Magnesium, Eisen, Zink oder Wolfram gehemmt, so dass die Korrosion temporär inhibiert ist.

Vorzugsweise ist R jeweils einzeln ausgewählt aus der Gruppe bestehend aus Methyl, Ethyl, Propyl und i-Propyl. Insbesondere enthält die Lösung Bis-[3-(triethoxysilyl)-propyl]tetrasulfid und/oder Bis-[3-(trimethoxysilylpropyl)]amin. Wird Bis-[3-(trimethoxysilylpropyl)]amine verwendet, so enthält die Lösung vorzugsweise Vinyltriacetoxysilan, zum Beispiel als 4:1 Mischung von Bis-[3-(trimethoxysilylpropyl)]amin und Vinyltriacetoxysilan, um den pH-Wert der Beschichtungslösung einzustellen. Nanopartikelhaltige Silanfilme als Korrosionsschutz für Aluminiumlegierungen wurden von V. Palanivel et al. bereits auf der Tagung ,The Workshop on Nanoscale Approaches for Multifunctional Coatings', Keystone, CO, USA, 12.-16. August 2002 vorgestellt. Vorzugsweise steht X ferner für eine C₂-C₁₀-Alkandiylgruppe (veraltet auch Alkylengruppe) oder eine substituierte oder unsubstituierte C₂-C₁₀-Heteroalkandiylgruppe mit 1 bis 3 Heteroatomen ausgewählt aus der Gruppe O, N und S.

Nach einer weiteren bevorzugten Ausführungsform des Verfahrens, die insbesondere auch in Kombination mit den zuvor genannten Varianten durchführbar ist, wird der Schritt (ii) des Beschichtens n-fach wiederholt, wobei n = 2 bis 10 ist und die in jedem Wiederholungsschritt eingesetzte homogen-kolloidale Lösung in Ihrer Zusammensetzung frei wählbar ist. Mit anderen Worten, die Beschichtung wird durch sequentielles Auftragen gleicher oder unterschiedlicher homogen-kolloidaler Lösungen erzeugt. Beispielsweise kann zunächst eine Bis-[3-(triethoxysilyl)-propyl]tetrasulfid-Lösung, dann eine Lösung, die Bis-[3-(trimethoxysilylpropyl)]amin und Vinyltriacetoxysilan enthält, und schließlich eine Bis-[3-(trimethoxysilylpropyl)]amin-Lösung verwendet werden.

Vorzugsweise ist der biokorrodierbare metallische Werkstoff eine biokorrodierbare Legierung ausgewählt aus der Gruppe der Elemente Magnesium, Eisen, Zink und Wolfram; insbesondere ist der Werkstoff eine biokorrodierbare Magnesiumlegierung. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Die Legierungen der Elemente Magnesium, Eisen, Zink oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden kathodischen, anodischen und chemischen Teilprozessen. Bei Magnesiumlegierungen ist in der Regel eine allmähliche Alkalisierung der Doppelschicht zu beobachten. Fremdstoffablagerungen, Verunreinigungen und Korrosionsprodukte beeinflussen den Korrosionsprozess. Die Vorgänge bei der Korrosion sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme. Dies gilt insbesondere für Stents, welche bei der Implantation lokal hohen plastischen Verformungen ausgesetzt sind. Konventionelle Ansätze mit starren korrosionshemmenden Schichten sind unter derartigen Voraussetzungen ungeeignet.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Die erfindungsgemäße korrosionshemmende Beschichtung kann im Laufe der Zeit selbst abgebaut werden bzw. kann die von ihr abgedeckten Bereiche des Implantats nur noch in einem immer geringeren Umfang schützen. Daher ist der Verlauf der Korrosionsrate für das gesamte Implantat nicht linear. Vielmehr ergibt sich zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate im Sinne der Erfindung verstanden und zeichnet die korrosionshemmende Beschichtung aus. Im Falle von Koronarstents soll die mechanische Integrität der Struktur über einen Zeitraum von drei bis sechs Monaten nach Implantation aufrechterhalten werden.

Ein weiterer Aspekt der Erfindung bezieht sich auf ein Implantat, das nach dem vorgenannten Verfahren herstellbar oder hergestellt ist. Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes, Okkluder und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtung ganz oder zumindest weitgehend diesen Anforderungen genügt.

Die zur Anbindung der Bis-Silane notwendigen Funktionalitäten können durch eine Vorbehandlung auf der Implantatoberfläche erzeugt werden, zum Beispiel durch Plasmabehandlung in sauerstoff- oder stickstoffreicher Atmosphäre oder ein kurzes Bad in Natronlauge.

Ein weiterer Aspekt der Erfindung betrifft ein Implantat aus einem biokorrodierbaren metallischen Werkstoff mit einer nanopartikel-haltigen Silanbeschichtung, wobei die Beschichtung
A) ein oder mehrere Bis-Silane der Formel (1):

   (RO)₃-Si-X-Si(OR)₃ (1)

   wobei R einzeln ausgewählt für C₁-C₁₀-Alkyl oder C₂-C₁₀-Acyl steht; und
   X eine substituierte oder unsubstituierte C₁-C₂₀-Alkandiylgruppe oder eine substituierte oder unsubstituierte C₁-C₂₀-Heteroalkandiylgruppe mit 1 bis 5 Heteroatomen ausgewählt aus der Gruppe O, N und S ist; und
B) Siliziumdioxid-Nanopartikel mit einem mittleren Partikeldurchmesser im Bereich von 10 nm bis 1 µm
umfasst.

Vorzugsweise ist R jeweils einzeln ausgewählt aus der Gruppe bestehend aus Methyl, Ethyl, Propyl und i-Propyl. Insbesondere ist das Bis-Silan Bis-[3-(triethoxysilyl)-propyl]tetrasulfid und/oder Bis-[3-(trimethoxysilylpropyl)]amin.

Vorzugsweise steht X ferner für eine C₂-C₁₀-Alkandiylgruppe oder eine substituierte oder unsubstituierte C₂-C₁₀-Heteroalkandiylgruppe mit 1 bis 3 Heteroatomen ausgewählt aus der Gruppe O, N und S.

Nach einer bevorzugten Ausführungsform des Implantats, weist das Implantat ein oder mehrere Schichten, gleicher oder unterschiedlicher Zusammensetzung, umfassend ein oder mehrere Bis-Silane der Formel (1) und Siliziumdioxid-Nanopartikel auf, insbesondere weist das Implantat *n* Beschichtungen auf, wobei *n* = 2 bis 10 ist.

Vorzugsweise ist der biokorrodierbare metallische Werkstoff eine biokorrodierbare Legierung ausgewählt aus der Gruppe der Elemente Magnesium, Eisen, Zink und Wolfram; insbesondere ist der Werkstoff eine biokorrodierbare Magnesiumlegierung. Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Vorzugsweise ist das Implantat ein Stent.

Die Erfindung wird nachfolgend anhand von Ausführungsbeispielen näher erläutert.

### Ausführungsbeispiel 1- Beschichtung eines Stents mit einem Bis-Sulfur-Silan

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Chloroform gewaschen, in Natronlauge (20%) zehn Minuten gebadet, mit deionisiertem Wasser gespült und getrocknet.

Es wurde eine 5 Vol. %ige-Lösung von Bis-[3-(triethoxysilyl)-propyl]tetrasulfid in einer wässrigen, ethanolischen Lösung angesetzt. Das Verhältnis des Bis-Silans zu Wasser und Ethanol betrug (5)/(5)/(90) Volumenanteile. Die Lösung wurde gerührt und bei Raumtemperatur für mindestens 48 Stunden belassen.

Es wurde weiterhin eine Suspension von SiO₂-Nanopartikeln mit einem mittleren Partikeldurchmesser von 15 Nanometern in Wasser bereitgestellt, wobei die erhaltene kolloidale Lösung 0,03 Gew.% der SiO₂-Nanopartikel enthielt.

Anschließend wurde eine Mischung aus 5 Teilen der kolloidalen nanopartikelhaltigen Lösung und 95 Anteilen des Bis-Silans hergestellt. Die Konzentration der Siliziumdioxidpartikel in der erhaltenen homogen-kolloidalen Silanlösung lag bei 15 ppm.

In diese Lösung wurde der Stent für 30 Sekunden getaucht, wieder entnommen, mit deionisiertem Wasser abgespült und bei 100°C eine Stunde getrocknet.

### Ausführungsbeispiel 2 - serielle Beschichtung eines Stents mit zwei Bis-Silanen

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Chloroform gewaschen, in Natronlauge (20%) zehn Minuten gebadet, mit deionisiertem Wasser gespült und getrocknet.

Es wurde eine 5 Vol. %ige-Lösung von einer 4:1 Mischung von Bis-[3-(trimethoxysilylpropyl)]amin und Vinyltriacetoxysilan in deionisiertem Wasser angesetzt. Die Lösung wurde gerührt und bei Raumtemperatur für mindestens 48 Stunden belassen.

Außerdem wurde eine 5 Vol. %ige-Lösung von Bis-[3-(triethoxysilyl)-propyl]tetrasulfid in einer wässrigen, ethanolischen Lösung angesetzt. Das Verhältnis des Bis-Silans zu Wasser und Ethanol betrug (5)/(5)/(90) Volumenanteile. Die Lösung wurde gerührt und bei Raumtemperatur für mindestens 48 Stunden belassen.

Es wurde weiterhin eine Suspension von SiO₂-Nanopartikeln mit einem mittleren Partikeldurchmesser von 1 µm in Wasser bereitgestellt, wobei die erhaltene kolloidale Lösung 0,03 Gew.% der SiO₂-Nanopartikel enthielt.

Anschließend wurde eine Mischung aus 5 Teilen der kolloidalen nanopartikelhaltigen Lösung und 95 Anteilen der jeweiligen Bis-Silan-Lösung hergestellt. Die Konzentration der Siliziumdioxidpartikel in der erhaltenen homogen-kolloidalen Silanlösung lag bei 15 ppm.

Die Beschichtung erfolgte seriell: 15 Sekunden in der nanopartikelhaltigen Bis-Sulfur-Silan-Lösung, anschließend 15 Sekunden in der nanopartikelhaltigen Bis-Amino-Silan-Lösung und wieder in der nanopartikelhaltigen Bis-Sulfur-Silan-Lösung für 15 Sekunden. Die Stents wurden dann mit deionisiertem Wasser abgespült und bei 100°C für zwölf Stunden getrocknet.

## Patentansprüche

1. Verfahren zur Herstellung eines Implantats aus einem biokorrodierbaren metallischen Werkstoff mit einer nanopartikel-haltigen Silanbeschichtung, wobei das Verfahren die Schritte umfasst:
(i) Bereitstellen eines Rohlings für das Implantat bestehend aus dem biokorrodierbaren metallischen Werkstoff; und
(ii) Beschichten des Rohlings mit einer homogen-kolloidalen Lösung aus
A) einem oder mehreren Bis-Silanen der Formel (1):
(RO)₃-Si-X-Si(OR)₃ (1)
wobei R einzeln ausgewählt für C₁-C₁₀-Alkyl oder C₂-C₁₀-Acyl steht; und
X eine substituierte oder unsubstituierte C₁-C₂₀-Alkandiylgruppe oder eine substituierte oder unsubstituierte C₁-C₂₀-Heteroalkandiylgruppe mit 1 bis 5 Heteroatomen ausgewählt aus der Gruppe O, N und S ist; und
B) Siliziumdioxid-Nanopartikeln mit einem mittleren Partikeldurchmesser im Bereich von 10 nm bis 1 µm
in Wasser, Ethanol oder einem Gemisch derselben, wobei die Lösung 1 bis 20 Vol.% Bis-Silan enthält und die Konzentration der Siliziumdioxid-Nanopartikel in der Lösung im Bereich von 1 bis 100 ppm liegt.

2. Verfahren nach Anspruch 1, bei dem der biokorrodierbare metallische Werkstoff eine biokorrodierbare Legierung ausgewählt aus der Gruppe Magnesium, Eisen, Zink und Wolfram ist.

3. Verfahren nach Anspruch 2, bei dem der biokorrodierbare metallische Werkstoff eine Magnesiumlegierung ist.

4. Verfahren nach einem der vorhergehenden Ansprüche, bei dem R jeweils einzeln ausgewählt ist aus der Gruppe bestehend aus Methyl, Ethyl, Propyl und i-Propyl.

5. Verfahren nach Anspruch 4, bei dem die homogen-kolloidale Lösung Bis-[3-(triethoxysilyl)-propyl]tetrasulfid und/oder Bis-[3-(trimethoxysilylpropyl)]amin enthält.

6. Verfahren nach Anspruch 4, bei dem die homogen-kolloidale Lösung eine Mischung aus Bis-[3-(trimethoxysilylpropyl)]amin und Vinyltriacetoxysilan enthält.

7. Verfahren nach einem der vorhergehenden Ansprüche, bei dem der Schritt (ii) n-fach wiederholt wird, wobei n = 2 bis 10 ist und die in jedem Wiederholungsschritt eingesetzte homogen-kolloidale Lösung in Ihrer Zusammensetzung frei wählbar ist.

8. Implantat aus einem biokorrodierbaren metallischen Werkstoff mit einer nanopartikel-haltigen Silanbeschichtung, wobei die Beschichtung
A) ein oder mehrere Bis-Silane der Formel (1):
(RO)₃-Si-X-Si(OR)₃ (1)
wobei R einzeln ausgewählt für C₁-C₁₀-Alkyl oder C₂-C₁₀-Acyl steht; und
X eine substituierte oder unsubstituierte C₁-C₂₀-Alkandiylgruppe oder eine substituierte oder unsubstituierte C₁-C₂₀-Heteroalkandiylgruppe mit 1 bis 5 Heteroatomen ausgewählt aus der Gruppe O, N und S ist; und
B) Siliziumdioxid-Nanopartikel mit einem mittleren Partikeldurchmesser im Bereich von 10 nm bis 1 µm
umfasst.

9. Implantat nach Anspruch 8, bei dem der biokorrodierbare metallische Werkstoff eine biokorrodierbare Legierung ausgewählt aus der Gruppe Magnesium, Eisen, Zink und Wolfram ist.

10. Implantat nach Anspruch 9, bei dem der biokorrodierbare metallische Werkstoff eine Magnesiumlegierung ist.

11. Implantat nach einem der Ansprüche 8 bis 10, bei dem das Implantat ein Stent ist.
